# EUROPEAN PATENT APPLICATION

(11) **EP 2 755 156 A2**
(43) Date of publication of application: **16.07.2014**
(21) Application number: 14150670.9
(22) Date of filing: 09.01.2014
(51) Int. Cl.: G06F 19/00

(54) **Techniques to improve accuracy of a medical report relating to a medical imaging study**

(30) Priority: 09.01.2013 US 201313737025; 09.01.2013 US 201313737039
(71) Applicant: Nuance Communications, Inc., Burlington, MA 01803 (US)
(72) Inventor: Mistry, Atul, North Andover, MA 01845 (US); Bauer, Jay Mark, Norcross, GA 30092 (US); Vadali, Manasavalli, Waltham, MA 02451 (US)
(74) Representative: Driver, Virginia Rozanne

(57) **Abstract**

Techniques for improving the accuracy of medical reports prepared by medical professionals. One technique may entail providing a system to automatically pre-populate a medical report by extracting data from a medical imaging study and pre-populating fields in the report with the extracted data. Another technique may entail providing a system to analyze data values from an imaging study during preparation of a medical report, and to generate alerts if at least one analyzed data value is outside of a normal range. The data extracted for pre-populating and/or the data values analyzed for alert generation may correspond to measurement data and/or calculation data from the imaging study. After pre-populating and/or generating alerts in the medical report, the system may present the report to a medical professional to include his/her impressions of the imaging study. Such techniques may enable medical professionals to more accurately and efficiently prepare medical reports for patients.

## Description

### CROSS-REFERENCE TO RELATED APPLICATIONS AND CLAIM OF PRIORITY

This application claims the benefit of priority of U.S. Patent Application No. 13/737,025, filed on January 9, 2013, and entitled "Techniques to Improve Accuracy of a Medical Report Relating to Medical Imaging Study," hereby incorporated by reference in its entirety. This application also claims the benefit of priority of U.S. Patent Application No. 13/737,039, filed on January 9, 2013 and entitled "Techniques to Improve Accuracy of a Medical Report Relating to a Medical Imaging Study," hereby incorporated by reference in its entirety.

### BACKGROUND

Medical imaging procedures of various types produce imaging studies that a medical professional may interpret to create a medical report. Examples of medical imaging procedures include High Technology Diagnostic Imaging (HTDI) (such as computed tomography (CT), magnetic resonance imaging (MRI), positron emission tomography (PET) and ultrasound imaging). An imaging study generated as a result of a medical imaging procedure typically contains several types of information related to the particular medical procedure performed on a patient. For example, such studies may contain information in the form of one or more images, videos, sounds, measurement data, and/or calculation data calculated from measurements taken from the image(s).

A medical professional typically analyzes the information in an imaging study and generates a medical report that includes the medical professional's impressions of the study and is associated with the patient's medical history. Such impressions can include the medical professional's opinions or interpretations of the patient's study (e.g., one or more diagnoses) and/or notes for possible follow-up tests, procedures and/or treatment. A medical professional typically enters such impressions in a medical report along with information from the medical imaging study.

### SUMMARY

One embodiment is directed to a computer implemented method of populating a medical report, the method comprising computer implemented steps of: extracting data from a memory, the memory holding data from a medical imaging study, the extracted data comprising measurement data from the medical imaging study and/or calculation data from the medical imaging study; and automatically populating at least one portion of the medical report with at least some of the extracted data, wherein the medical report comprises a medical professional's impression of the medical imaging study.

The step of extracting data from the medical imaging study may comprise at least one of extracting measurement data from the medical imaging study, wherein the measurement data comprises data collected by at least one medical imaging device; and extracting calculation data from the medical imaging study, wherein the calculation data comprises data calculated based, at least in part, on data collected by at least one medical imaging device.

The computer implemented method may comprise automatically determining a type of the medical imaging study; and automatically determining what data to extract from the medical imaging study based, at least in part, on the determination of the type of medical imaging study.

The medical imaging study may comprise a DICOM Structured Report (SR) comprising binary data, and wherein the extracting data from the medical imaging study may comprise converting the binary data into XML-formatted data and extracting the data from the XML-formatted data.

In one embodiment, the at least one portion of the medical report may be associated with a respective identifier identifying the type of data to be populated in said at least one portion, and the step of automatically populating a portion of the medical report may comprise determining a data type of a portion of the extracted data; and populating the portion of the medical report with said portion of the extracted data based on determining that the data type identified by the identifier associated with the portion of the medical report matches the data type of the portion of extracted data.

In one embodiment the at least one portion of the medical report is a merge field, the method comprising using a merge field module to locate and extract the data when said merge field is referenced in the medical report and automatically populating the merge field with the extracted data.

The computer implemented method may further comprise analyzing at least one value of at least one data type from the medical imaging study, the at least one data type comprising at least one type of measurement data from the medical imaging study and/or at least one type of calculation data from the medical imaging study; determining whether the at least one analyzed data value is outside of a normal range for the at least one data type; and during preparation of the medical report, generating an alert to the medical professional in response to determining that the at least one analyzed data value is outside of the normal range.

The extracted data may comprise the at least one analyzed data value.

The at least one analyzed data value may not be among the extracted data.

Another embodiment is directed to a system configured to populate a medical report.

The system comprises at least one processor and at least one storage device storing computer-readable program instructions. The stored computer-readable program instructions, when executed by the at least one processor, performs any of the method steps described above.

One embodiment is directed to a computer implemented method comprising computer implemented steps of analyzing at least one value of at least one data type from a medical imaging study held in a memory, the at least one data type comprising at least one type of measurement data from the medical imaging study and/or at least one type of calculation data from the medical imaging study; determining whether the at least one analyzed data value is outside of a normal range for the at least one data type; and during preparation of a medical report comprising a medical professional's impression of the medical imaging study, generating an alert to the medical professional in response to determining that the at least one analyzed data value is outside of the normal range.

The step of analyzing at least one value of at least one data type may comprise at least one of analyzing measurement data from the medical imaging study, wherein the measurement data comprises data collected by at least one medical imaging device; and analyzing at least one value of at least one data type comprises analyzing calculation data from the medical imaging study, wherein the calculation data comprises data calculated based, at least in part, on data collected by the at least one medical imaging device.

The medical imaging study may relate to a patient, and wherein the normal range may comprise a range of values corresponding to a determined normal range for a population comprising a plurality of subjects, and/or a determined normal range for the patient.

The computer implemented method may comprise automatically determining a type of the medical imaging study; and automatically determining the at least one value of the at least one data type to analyze based, at least in part, on the determination of the type of medical imaging study.

The computer implemented method may further comprise extracting data from the medical imaging study, the extracted data comprising measurement data from the medical imaging study and/or calculation data from the medical imaging study; and automatically populating at least one portion of the medical report with at least some of the extracted data, wherein the medical report comprises the medical professional's impression of the medical imaging study.

Another embodiment is directed to a system. The system comprises at least one processor and at least one storage device storing computer-readable program instructions. The stored computer-readable program instructions, when executed by the at least one processor, performs any of the method steps described above.

Another embodiment is directed to a computer program product comprising a computer readable medium having stored thereon computer-readable program instructions. The stored computer-readable program instructions, when executed by at least one processor, performs any of the method steps described above.

It should be appreciated that all combinations of the foregoing concepts and additional concepts discussed in greater detail below (provided that such concepts are not mutually inconsistent) are contemplated as being part of the inventive subject matter disclosed herein.

### BRIEF DESCRIPTION OF DRAWINGS

The accompanying drawings are not intended to be drawn to scale. For purposes of clarity, not every component may be labeled in every drawing. In the drawings:
FIG. 1 is a schematic illustration of a computing environment in which techniques may be used to improve the accuracy of a medical report, in accordance with some embodiments;
FIG. 2 is a schematic illustration of an illustrative computing system that may be used to improve the accuracy of medical report, in accordance with some embodiments;
FIG. 3 is a sketch of an illustrative medical report containing pre-populated data and alerts, in accordance with some embodiments;
FIG. 4 is a flowchart of an illustrative method of extracting data from an imaging study and pre-populating a medical report, in accordance with some embodiments;
FIG. 5 is a flowchart of an illustrative method of analyzing data from an imaging study and generating an alert during the preparation of a medical report, in accordance with some embodiments; and
FIG. 6 is a schematic illustration of a representative computing device that may be used to implement techniques to improve the accuracy of a medical report, in accordance with some embodiments.

### DETAILED DESCRIPTION

The inventors have recognized and appreciated that accuracy is critical in the preparation of medical reports. A medical professional preparing a medical report on a medical imaging study may examine various information from the study to formulate an impression that is memorialized in the medical report. In addition to entering his/her impressions in the medical report, the medical professional may also enter in the medical report various data (e.g., measurement data and/or calculation data) from the imaging study.

The inventors have recognized and appreciated that manual input of data into a medical report may be prone to errors. Such errors may cause medical professionals accessing the medical report to have inaccurate information which may have potentially serious consequences (e.g., misdiagnosis, mistreatment, etc.). In addition, verifying the correctness of data entry in a medical report may be costly, both in terms of time and manpower. Such challenges may be exacerbated for studies that generate large amounts of data, such as medical imaging studies.

The inventors have also recognized and appreciated that given the voluminous data in an imaging study, accurately analyzing a patient's study may be a challenge, and may result in a medical professional failing to identify one or more abnormalities in a patient's study. This may be true while a medical professional is generating the medical report and formulating his/her impressions to be included therein, and may then be perpetuated by the abnormalities going undetected by medical professionals who later access the medical report.

In accordance with some embodiments, techniques are provided to improve the accuracy of medical reports resulting from an imaging study. In accordance with some embodiments described herein, techniques for populating a medical report are provided. Measurement data and/or calculation data from the medical imaging study are extracted, and at least one portion of the medical report is automatically populated with at least some of the extracted data. In this manner, the medical report, which comprises a medical professional's impression of the medical imaging study, is ensured to have accurate data included therein, as the data is extracted and automatically populated from the medical imaging study.

In accordance with some other embodiments described herein, at least one value of at least one data type (e.g., measurement data and/or calculation data) from a medical imaging study is analyzed, a determination is made as to whether the analyzed data value is outside of a normal range for the corresponding data type, and then during preparation of a medical report comprising a medical professional's impression of the medical imaging study, an alert is generated to the medical professional in response to determining that at least one analyzed data value is outside of the normal range.

The determination of a normal range of values and/or whether a value falls outside of a normal range may be made in any suitable way. For example, a normal range may be determined based on established governmental and/or industry standards. Alternatively or additionally, a normal range of values may be determined based on statistical sampling of a group of subjects, historical data of the subject of the medical imaging study, and/or in any other suitable way. Regardless of how the normal range is determined, it may indicate a set of values that is considered "normal" or "healthy" for a patient, according to any suitable criteria.

In some embodiments described below, these techniques can be used together. However, it should be appreciated that all embodiments described herein are not limited in this respect, and that each of these concepts can alternatively be used separately.

Some illustrative embodiments described below are described in connection with the processing of medical imaging studies that are formatted in accordance with the Digital Imaging and Communications in Medicine (DICOM) standard, which is a known standard for formatting and communicating data from an imaging study. The results of an imaging study in accordance with the DICOM standard are provided in a number of DICOM objects. The DICOM objects include numerous types of data that result from a medical imaging study and provides a standardized format for organizing and representing that data. The specific data may vary depending upon the particular type of medical imaging study that generated the DICOM objects (e.g., an MRI, a CT scan, an ultrasound, etc.) but examples of data that may be included in DICOM objects include image data, video data, acoustic data and measurement and/or calculation data. The measurement data and/or calculation data are stored in a type of DICOM object referred to as a DICOM Structured Report (SR) and can be generated in any suitable manner. For example, a technician that operates the imaging system when performing a medical imaging study can take one or more measurements of objects appearing in the image (e.g., body parts, tumors, a fetus, etc.) and those measurements can be included in the DICOM Structured Report. Calculation data can be data that results from performing any type of calculation on measurement data to generate a calculated value that is derivable from one or more measurements taken.

Although medical imaging studies in a DICOM Structured Report are described herein as one illustrative example, it should be appreciated that the techniques described herein are not limited to use with DICOM Structured Reports, and can be used with medical imaging studies that are provided in any suitable format.

In some embodiments described herein, techniques are provided to improve accuracy of a medical report relating to a medical imaging study. The medical report can be created by any suitable medical professional accessing the medical imaging study in any suitable way. An example of such a medical professional is a radiologist reviewing an imaging study (e.g., an MRI or CT scan) and preparing a medical report based thereon. However, this is merely one example, as the techniques described herein can be used to facilitate the generation of the medical report by any medical professional.

In accordance with some embodiments, the techniques described herein can be used in connection with systems that are specially designed to facilitate the generation of a medical report by a medical professional reviewing the results of an imaging study. An example of such a system is the PowerScribe™ product available from Nuance Communications, Inc. Such systems allow a medical professional to review images from the imaging study on one monitor screen, review other types of data (e.g., measurement data) from the imaging study on another screen, and have a third screen that provides a user interface to a tool or application that facilitates the generation of a medical report relating to the imaging study. The PowerScribe™ system is described merely as one example, as the techniques described herein are not limited to use with the PowerScribe™ system or any other system specifically designed to facilitate the generation of a medical report from a medical imaging study.

The system may include a user interface that facilitates medical report generation and enables a medical professional to enter his/her impressions of the medical study. For example, the user interface may enable the medical professional to dictate observations, and may include an automatic speech recognition (ASR) engine that transcribes the dictation. In addition, or alternatively, the interface may enable the medical professional to use a keyboard, mouse, touchscreen and/or any other suitable data entry device. It should be appreciated that the techniques described herein can be used with systems that are not limited in any way by the nature of the user interface used by the medical professional to generate a medical report.

In accordance with some illustrative embodiments described below, the techniques described herein can be employed in systems that support large institutions with a large number of imaging studies being interpreted by a large number of different medical professionals. In such systems, the medical imaging studies may be stored in one or more centralized data repositories and then distributed to a number of remote computing systems where they may be accessed by medical professionals generating medical reports relating thereto. The centralized repository may be implemented using one or more servers that may make the imaging studies available to remote computing systems via the Internet, an intranet or using any other suitable communication.

Although this type of large institutional system is described in connection with some embodiments below, it should be appreciated that techniques described herein are not limited to use in such systems, and can be employed with any type of system in which a medical professional has access to an imaging study (whether stored locally on the imaging system that generated the imaging study or on another computer accessible to the medical professional or otherwise), as the techniques described herein are not limited to any particular type of system.

For example, some large institutions have a Picture Archiving and Communication Systems (PACS) database from which imaging studies may be retrieved by a client computer operated by a medical professional. Alternatively, in some smaller institutions, a medical professional may access an imaging study directly from the imaging device itself, which may store the imaging study in the DICOM standard or in any other format. When the imaging study data is stored on a computer remote from the medical professional, the system that stores the medical imaging data may include a web interface that enables the medical professional to access the imaging study data using a browser on the medical professional's computer. Again, the specific examples of systems in which the techniques described herein can be used are provided merely for illustrative purposes, as the techniques described herein are not limited to use with any particular type of system.

In accordance with some embodiments described below, techniques described herein can be employed with systems that include templates for different types of medical imaging studies. The templates can be used by a medical professional to facilitate the preparation of a particular type of medical report. In accordance with some embodiments described herein, when a medical professional calls up a particular type of medical report (e.g., an imaging study relating to the left shoulder), the medical professional is initially provided with a template report that conforms to the template and includes a number of fields that correspond to the information to be provided in the corresponding type of medical report. The medical professional can then provide additional information into the template report which can then be finalized to create the medical report.

In accordance with some embodiments described herein, some fields in a template may relate to data that may be extracted directly from the medical imaging study (e.g., measurement data, calculation data, etc.). In such circumstances, in accordance with some embodiments described herein, one or more of those fields may be automatically populated by the system by extracting the data directly from the medical imaging study. The fields in a template may be associated with a respective identifier which identifies the type of data that is to be populated in the respective field. An item of data can be automatically populated into a field by determining the type of data of the item of data and matching this data type to a field based on the field's identifier. In this manner, when the medical professional begins the process of preparing a particular type of medical report using an associated template, when the medical professional calls up the template report, one or more fields of the template report are already populated. In this manner, accuracy is improved, as the medical professional need not manually input the data from the medical imaging study into the appropriate fields in the template medical report. In addition, a time savings may be achieved in the preparation of the medical report, as the medical professional need not take time to manually input data from the imaging study into appropriate fields in the template medical report.

As discussed above, reports for different types of studies may take different forms, and an appropriate template may be created for any and/or all types of reports. In addition, in accordance with some embodiments described herein, if the imaging study (e.g., in a DICOM Structured Report or other format) includes measurement data and/or calculation data that is relevant to the particular type of medical report and the template that was previously created therefor, that data is automatically extracted from the medical imaging study and the corresponding fields in the medical report are prepopulated with that data. In accordance with one embodiment, a set of procedure codes is established that identifies various types of medical imaging studies, as well as the reports and corresponding templates related thereto.

As discussed in greater detail below, in some embodiments the concept of a merge field is employed to facilitate the generation of templates in a manner that is generic to imaging studies that take different formats (e.g., from different vendors) and to modularize the generation of templates so that one or more merge fields can be used in multiple different types of templates. As one example, multiple types of medical reports may include a field for the patient's heart rate. Rather than writing separate software code for every template to provide it with the ability to access imaging studies of all different types (e.g., from different vendors) and to locate and extract the data representing the patient's heart rate from the imaging study, a merge field can be created for this purpose. Thus, each template that has a field relating to the patient's heart rate may simply include a reference to the common merge field. A single merge field macro or module can then be created (e.g., by training on data samples from all suitable types of imaging studies, through the creation of rules that specify where the relevant data can be found in all types of imaging studies, etc.) and can be used each time the relevant merge field is referenced in a template. While merge fields provide the advantages described above, it should be appreciated that all embodiments described herein are not limited to their use, as the techniques described herein are not limited to systems that employ merge fields.

As discussed above, in some embodiments, at least one value of at least one data type from a medical imaging study can be analyzed to determine whether it is outside of a normal range, and if so, an alert can be generated to the medical professional during the preparation of a medical report relating to the medical imaging study. Data values that are outside of the normal range may indicate an abnormality in the patient's study that may, if recognized by the medical professional, influence the diagnosis, treatment plan or other impressions of the medical professional. Thus, by alerting the medical professional (e.g., in any of numerous ways, examples of which are discussed below) to the data value being outside of its normal range, the likelihood that the medical professional will overlook the fact that the data value is outside of its normal range is greatly diminished, thereby increasing the accuracy of the medical report by ensuring that any impressions of the medical professional included therein result from properly recognizing that the data value falls outside of its normal range.

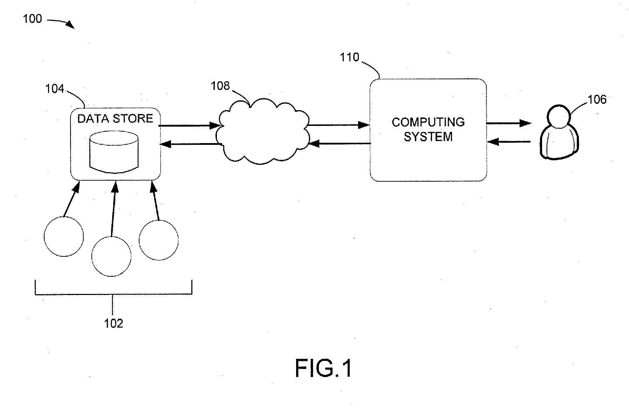

FIG. 1 illustrates an illustrative computing environment 100 in which the techniques described herein can be employed. In the computing environment 100, imaging studies from a plurality of medical imaging systems 102 are stored in a data store 104. As discussed above, the medical imaging systems 102 can take any of numerous forms, as the techniques described herein are not limited to use with any particular type of medical image systems. Similarly, the medical imaging data and data store 104 can take any suitable form, as the techniques described herein are not limited in this respect. In some embodiments, medical imaging studies conform to the DICOM format and the data store 104 may be a DICOM-compliant data store, such as a Picture Archiving and Communications System (PACS) database, but these are merely examples, as the techniques described herein are not limited to use with medical imaging studies according to the DICOM standard nor to the use of a PACS database.

In connection with embodiments in which the imaging study data may be stored in accordance with the DICOM format, it should be appreciated that DICOM is a binary format, and a computing system capable of processing image reports according to the DICOM format may have the ability to interpret them. As discussed above, the imaging study may include any one or more of various types of data, including image data, sound data, video data, etc. In accordance with the DICOM standard, each type of data may be formatted as a separate DICOM object, and the object may include a header identifying the type of data stored therein and optionally some information that may be used to decode it. In addition, a DICOM Structured Report object may include any of various types of measurement data and/or calculation data.

In accordance with some embodiments, the data store 104 may include one or more computers (e.g., servers) that may have some ability to route image study data to one or more interested parties seeking access to them. As an example, for embodiments in which PACS is used, the data store may include a PACS server that has such a capability, although the techniques described herein are not limited to use with PACS.

In the illustrative system of FIG. 1, an example of a party interested in analyzing an imaging study stored on the data store 104 is shown in the form of a medical professional 106 who may access a medical imaging study via a computing system 110. The computing system 110 is coupled to the data store 104 via a communication medium 108. The communication medium 108 may be a wide area network (e.g., the Internet), an intranet, or any other suitable communication medium or combination of communication media, as the techniques described herein are not limited in this respect. Similarly, the computing system 110 may be a desktop or workstation specifically programmed to facilitate the reading of medical imaging studies (examples of which were described above), but the techniques described herein are not limited in this respect and can be used with any computing system capable of accessing a medical imaging study.

The distribution of medical imaging studies from the data store 104 to one or more computing systems 110 can be performed in any suitable way, as the techniques described herein are not limited in this respect. In accordance with some embodiments wherein the data store 104 may serve a large institution with a large number of medical professionals who may potentially be seeking access to different imaging studies, techniques can be employed to ensure that privacy concerns are addressed. This can be done in any suitable way, as the techniques described herein are not limited in this respect. Furthermore, as discussed above, the techniques described herein are not limited to use in a distributed system wherein a data store 104 is accessible to a large number of medical professionals by various computing systems 110, and can be employed in systems wherein the medical professional can access the medical imaging study data directly from the imaging device, such as any of the imaging devices 102.

In accordance with some embodiments described herein, software that may reside on the computing system 110 may register as a recipient of medical imaging study data and have the ability to interpret the medical imaging study data. For example, when the medical imaging study data is stored in the DICOM format, the software may register as a recipient of DICOM objects and have the ability to interpret them. When a medical professional (e.g., the medical professional 106 in FIG. 1) uses the computing system 110 to put in an order requesting access to a particular medical imaging study (which may be identified in any suitable manner, as the techniques described herein are not limited in this respect), in addition to presenting to the medical professional 106 the data from the medical imaging study (which may be done in any suitable way, examples of which are discussed above), a template report is created for the study.

It should be appreciated that the processing of image study data to facilitate the preparation of the medical report can be done in any suitable manner, as the techniques described herein are not limited in this respect. In accordance with one embodiment of the present invention, the computing system 110 may register with the data store 104 to receive imaging studies that will be accessed by one or more medical professionals using the computing system 110. This can be done at any level of granularity, including for individual medical professionals, for entire medical institutions (hospitals, medical practices, etc.) or in any other suitable way. The medical imaging studies may be forwarded to the computing system 110 when a medical professional (e.g., medical professional 106 in FIG. 1) requests access to it. However, in accordance with other embodiments, the medical imaging studies may be sent to the computing system 110 once they have been completed, so that more efficient local access can be provided when a medical professional seeks to access a medical imaging study.

In accordance with some embodiments of the present invention, when a medical imaging study is received at the computing system 110, some pre-processing can be performed on it, although the techniques described herein are not limited in this respect, as all processing can await a request from a medical professional to access the medical imaging study. One example of pre-processing that can be performed relates to medical imaging studies formatted according to the DICOM standard, wherein the imaging study is provided in binary format. In accordance with one embodiment described herein, the binary DICOM objects can be pre-processed and converted to a different format (e.g., XML) and stored in a data store managed by the computing system 110 to await access by one or more medical professionals. It should be appreciated that is simply one example of pre-processing, and that the embodiments described herein are not limited in this respect.

When a medical professional seeks to access the medical imaging study via the computing system 110, the medical imaging study data (which may have been pre-processed as discussed above, may be stored locally by the computing system 110 in precisely the manner in which it was received from the data store 104, which may come directly from the data store 104 in response to the medical professional seeking access, etc.) may be examined to extract any data relevant to the associated template report so that one or more fields can be pre-populated with that data in the manner discussed above.

It should be appreciated that the templates supported by the computing system 110 and any associated procedure codes may be specified earlier, (e.g., during initialization of the system). Initialization can include defining the relevant templates, identifying them as corresponding to the appropriate procedure codes, defining any desired merge fields, specifying any rules and/or models that inform from where and how relevant data is to be extracted for each field (e.g., a merge field or otherwise) that may be pre-populated, or any other suitable initialization action(s).

As discussed above, in accordance with other embodiments, during the preparation of a medical report by a medical professional (e.g., the medical professional 106 in FIG. 1), at least one value of at least one data type from the corresponding medical imaging study can be analyzed to determine whether it is outside of a normal range, and when it is determined that at least one analyzed data value is outside of the normal range, an alert can be generated to the medical professional to increase the likelihood that the medical professional will not overlook the abnormal data value(s). This can be performed in any of numerous ways, as the techniques described herein are not limited to any particular way of generating alerts.

In accordance with one embodiment, the above-described software component that receives a medical imaging study, creates a corresponding report template and pre-populates fields thereof can also perform the analysis and generate an alert to the medical professional. However, it should be appreciated that the techniques described herein are not limited in this respect, and that the analysis and/or alert generation can be done by one or more other software components (whether operating on a computing system such as computing system 110 that is local to the medical professional or a computer system remote therefrom). The analysis and alert generation can be performed on data values that are provided in the fields of the medical report, and/or on any other data values in the medical imaging study, as the techniques described herein are not limited in this respect.

While the techniques described herein for automatically populating at least a portion of a medical report with data extracted from a medical imaging study and generating an alert for data values outside of a normal range can be used together in some embodiments, it should be appreciated that all embodiments are not limited in this respect, and that in some embodiments, only one of these techniques can be employed without the other. For example, in some embodiments, some portions of a medical report may be pre-populated with data from a medical imaging study but no alert generation capability may be provided. Alternatively, in other embodiments, an alert generation capability may be provided, but no automatic population of any portion of a medical report may be performed.

It should be appreciated that the analysis of data values in the medical imaging study for the purpose of determining values outside of the normal range can be performed in any suitable way, as the techniques described herein are not limited to any particular implementation technique. For example, in accordance with one embodiment described herein, for each type of medical report, a set of rules may be written that specify the data values to be analyzed and the value ranges that will result in the generation of an alert. In accordance with other embodiments, one or more statistical models may be trained using a set of training data and the statistical model(s) can evaluate the data in a medical imaging study for values outside of an expected range either instead of or in addition to the set of rules as discussed above. It should be appreciated that in accordance with some embodiments described herein, the alert can be binary, in that an alert is either generated or it is not. However, the techniques described herein are not limited in this respect, as in other embodiments alerts may be generated with greater degrees of granularity such that different types of alerts (e.g., using different visual techniques such as colors or fonts on a medical report, different types of audible tones, etc.) can be used for data values that fall outside of the expected range in different ways or by different degrees.

Alerts may be generated in any suitable manner, as the techniques described herein are not limited to any particular way of alerting the medical professional to an abnormality in the data in the medical imaging study. As some non-limiting examples, alerts can be generated audibly and/or visually. For example, when an alert relates to a data value that is populated within a field of the medical report template, that field may be visually indicated to the medical professional, using color, highlighting, font size or type, emphasis (e.g., bolding, italics, underlining, etc.) or in any other suitable way to make the data values stand out from others in the medical report template. As discussed above, the analyzing of data in an imaging study for the purpose of generating an alert is not limited to analyzing data included in fields in the medical report, as in some embodiments any and all data in an imaging study may be analyzed for abnormalities and one or more alerts may be generated based thereon.

The computing system 110 may be implemented using one or more computing devices. In some embodiments, computing system 110 may comprise a client-server configuration. For example, one or more servers may receive and store imaging data, and the data may be accessed by multiple client workstations used by different medical professionals. A client-server configuration may be used, for example, in an institutional setting, (e.g., a large hospital or health care organization) where medical imaging studies may be received at one or more servers for the institution and then be accessed in a distributed manner from different locations by medical professionals affiliated with the institution. It should be appreciated, however, that computing system 110 is not limited to a client-server configuration, and that computing system 110 may comprise a single computing device, or two or more computing devices that distribute processing to perform the techniques described herein in any suitable manner.

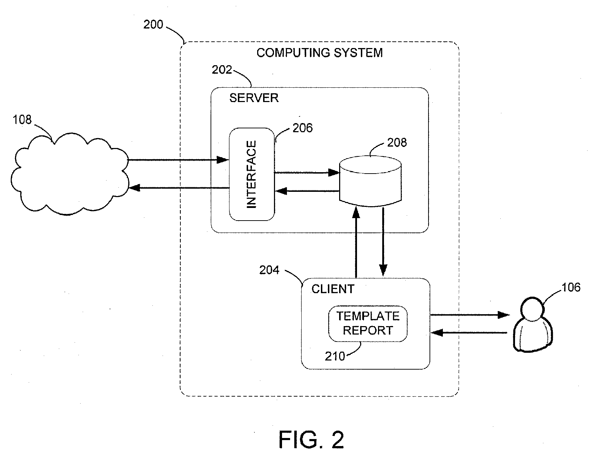

A non-limiting example of a client-server configuration that can be used to implement computing system 110 is illustrated in FIG. 2. A computing system 200 comprises a server computing device 202 and a client computing device 204. The server device 202 may receive imaging study data from communication medium 108, and distribute imaging study data to one or more client devices 204 that may use the data to facilitate preparation of a medical report by a medical professional 106 in any of the ways discussed above. In some embodiments, the DICOM standard may be used to format and communicate the imaging data. It should be appreciated, however, that the techniques described herein are not limited to using DICOM, as any suitable technique may be used to format and communicate medical imaging data.

The server device 202 has an interface 206 for receiving imaging data study from the communication medium 108. The interface 206 may be configured to handle any particular communication protocol(s), as the techniques described herein are not limited in this respect. For example, to support the DICOM standard, the interface 206 may be configured to implement a network service for receiving a DICOM SR object from a DICOM server or router over a TCP connection. However, it should be appreciated that the interface 206 may be configured to receive any suitably-formatted imaging data using any suitable communication protocol.

In some embodiments, after receiving a DICOM SR object, the interface 206 may convert the DICOM SR into the Extensible Markup Language (XML) or another data format for storage in a local data store 208. It should be appreciated, however, that converting the received data into another format is optional, as in some embodiments, the received data may be stored in data store 208 in the format in which it is received.

In some embodiments, the server device 202 may attempt to correlate the received DICOM SR object with one or more existing orders that a medical professional may have put in to request the imaging study. If a matching order is found, then the server device 202 may save the DICOM SR object in the data store 208 as an attachment to the corresponding order(s). Otherwise, the server device 202 may create a temporary order and associate the DICOM SR object with the temporary order.

The medical professional 106 may begin preparing a medical report by opening an order using the client device 204. If no order was previously identified in the system for an imaging study so that the imaging study is stored in a temporary order, the medical professional may use the client device 204 to examine information about temporary orders to identify the appropriate one. The client device 204 may load the requested order from the data store 208. If there is DICOM data associated with the requested study, then the retrieved order may include, as an attachment, a DICOM SR object. In some embodiments, the client device 204 may extract relevant data from the received DICOM SR object, and pre-populate a template medical report 210 as discussed above.

In some embodiments, the template report 210 may include various placeholders for data. For example, such placeholders may be fields (which may be merge fields as discussed above), and each field may correspond to a particular type of data in the DICOM SR object. For example, a template medical report 210 may have separate merge fields for different types of data, such as heart rate, bone density, blood flow, and/or other data collected by the medical imaging procedure(s). The client device 204 may pre-populate one or more fields in the template report 210 by extracting the corresponding data from the DICOM SR object.

In addition, or as an alternative, to extracting data from a DICOM SR object and prepopulating the template report 210, the client device 204 may also implement other techniques to improve the accuracy of the resulting medical report. In some embodiments, the client device 204 may analyze data in the DICOM SR object and determine whether any data values are outside of a normal range of values. Based on this determination, the client device 204 may generate an alert to the medical professional 106.

The client device 204 may present the template report 210 (which may be pre-populated with data in some embodiments) to the medical professional 106. The template report can be considered to be a preliminary report. The medical professional 106 may then input his/her impressions of the study into the template report 210 and sign off on the report to complete the final report.

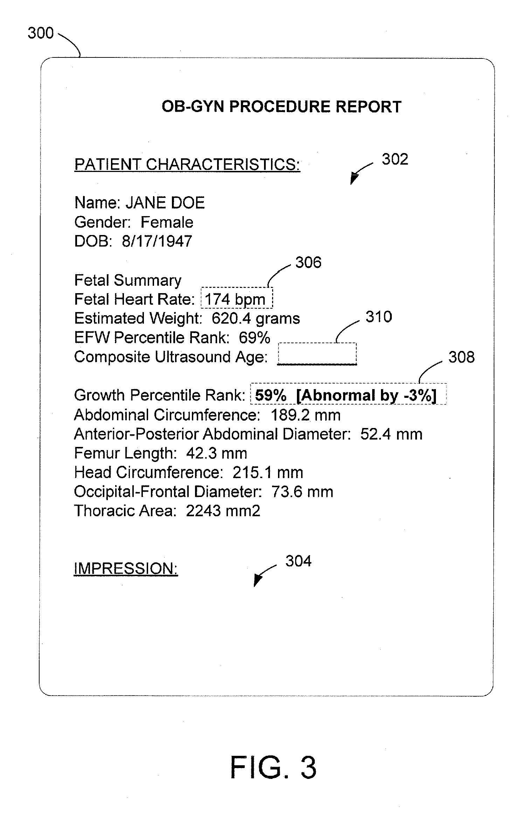

An example of a template medical report 300 is illustrated in FIG. 3. Template medical report 300 corresponds to a study resulting from an OB-GYN procedure. The template report 300 comprises a first section 302 that presents various types of data identifying the patient and data collected from the imaging study, and a second section 304 in which a medical professional may record his/her impression(s) of the study.

In some embodiments, the first section 302 may be pre-populated with data from the imaging study. As non-limiting examples, a Fetal Heart Rate field is pre-populated at 306 and a Growth Percentile Rank field is pre-populated at 308. These are merely examples, as the pre-populated data may be of any of a variety of different types, examples of which include, but are not limited to, numeric values (with units), patient identifying information, dates and times, plain text notes, coded values, waveforms and other composite objects or spatial and/or temporal coordinates in referenced objects.

As discussed above, the fields 306 and 308 that are pre-populated may be merged fields when they are used in multiple different types of templates, or they may be fields that are specific to only one template. In the illustrative medical report shown in FIG. 3, a field 310 corresponding to Composite Ultrasound Age is left unpopulated. This may be for any of numerous reasons. For example, when the template was created, intelligence (e.g., rules, models, etc.) that would inform the field 310 of how and where to find the corresponding data in a medical imaging study may not have been provided. Alternatively, the particular medical imaging study may lack the data necessary to determine a value for the particular field.

In addition or as an alternative to pre-populating fields of the template medical report 300 with data, one or more alerts may be generated for the medical report 300 when it is determined that one or more data values is outside of a normal range. In FIG. 3, an alert is shown for the Growth Percentile Rank field 308. Any suitable technique may be used for presenting alerts. As examples, the field 308 may be highlighted, may be in a different color (such as red), may be bolded (as shown in FIG. 3), or other techniques may be used. Alerts may be audible, in addition or as an alternative to being visual. For example, audible alerts may include a sound (e.g., a bell, chime), or a recorded or synthesized voice presenting information (e.g., a standard message or more detailed information about the particular detected abnormality), or any other suitable audible alert that can be presented to the medical professional during preparation of the medical report.

In the particular example shown in FIG. 3, the alert not only identifies a value that is outside of the normal range, but also quantifies an amount by which the value is abnormal by stating that the amount is "Abnormal by -3%." It should be appreciated that the techniques described herein are not limited to generating alerts that quantify an amount by which a data value is abnormal by using a percentage, or in any other way, as an alert could alternatively simply identify a data value that is outside of the normal range without quantifying the degree or amount of abnormality.

The normal range may be determined in any suitable way. In the example of FIG. 3, a normal range for Growth Percentile may be determined based on a range of fetal measurements certified by a medical organization, such as the Board of Radiology, as being "normal" and/or may be determined based on statistical sampling of fetal measurements. Regardless of how the normal range(s) of values is determined, in some embodiments, an alert indicating one or more data values outside of the expected normal range(s) may indicate an unhealthy condition of the patient.

The medical professional may formulate one or more impressions of the patient's study, and enter the impression(s) in the second section 304 in any suitable way. The impression(s) may comprise opinions of the medical professional based on analyzing and/or interpreting the medical imaging study.

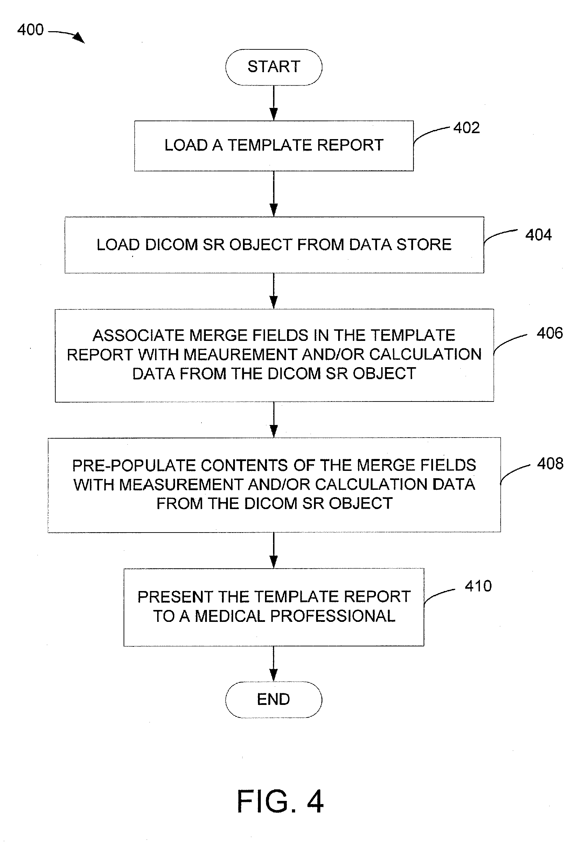

FIG. 4 is a flowchart of an exemplary process 400 for extracting data from an imaging study and pre-populating a template medical report, in accordance with some embodiments. Process 400 may be implemented in any suitable way. For example, process 400 may be implemented in software that can run on any suitable computer system, an example of which is a workstation accessed by medical professionals to prepare medical reports (e.g., client computer 204 in FIG. 2). Process 400 may be initiated in response to a request from a medical professional to prepare a medical report for a particular study.

In step 402, a template report may be loaded, corresponding to the type of study that is requested. In step 404, the image study data (e.g., in the form of a DICOM SR object or other form) is loaded from a data store (e.g., data store 208 of FIG. 2) in a server.

After the image study data is loaded from the data store, the relevant data in the image study data (e.g., the DICOM SR object) may be associated, in step 406, with the corresponding fields in the template report. This can be done in any suitable way, examples of which are discussed above (e.g., having one or more rules and/or models for each field of a template capable of being pre-populated).

Then, the corresponding data values may be used to populate the corresponding fields in the template report, in step 408. After the fields in the template report have been populated with data from the imaging study data (e.g., DICOM SR object), the pre-populated template report may be presented to the medical professional, in step 410. The medical professional may then enter other information into the template medical report, including his/her impressions of the study.

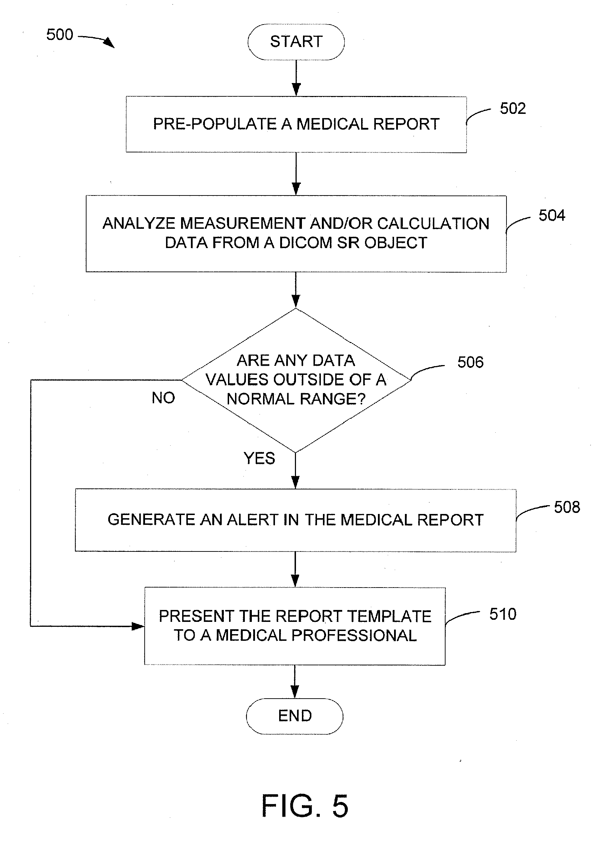

FIG. 5 is a flowchart of an exemplary process 500 of analyzing data from an imaging study and generating an alert to a medical professional preparing a medical report, in accordance with some embodiments. Process 500 may be implemented in any suitable way. For example, process 500 may be implemented in software that can run on any suitable computer system, an example at which is a workstation (e.g., client computer 204 in FIG. 2) used by the medical professional in preparing the medical report. The process 500 may be implemented either in conjunction with, or as an alternative to, the process 400 of FIG. 4. Process 500 may be initiated in response to a request from a medical professional to begin preparing a medical report, in response to a request to check the image study data for abnormalities, or in any other suitable way.

In some embodiments, in step 502, the workstation may pre-populate the medical report (e.g., using the process 400 of FIG. 4). However, it should be appreciated that process 500 may be implemented without pre-populating the medical report.

In step 504, the image study data may be analyzed to detect one or more anomalies. Anomalies may be, for example, data values that are outside of a normal range of values. A normal range of values may depend on several factors, such as the type of data being considered, the type of study, etc. The normal range may be particular to an individual patient, an aggregate of a sample of patients, a combination of both, or may be determined in any other suitable way, as the techniques described herein are not limited in this respect. Regardless of how a normal range is determined, the image study data may be analyzed to detect whether one or more data values is outside of its normal range.

In step 506, if no data value is found to be outside of the normal range, then the template report may be presented to the medical professional, either unpopulated or pre-populated with data from the image study data. If, however, one or more data values are found to be outside of their normal ranges, then in step 508, an alert may be generated. The alert may be presented, for example, in the medical report itself. However, it should be appreciated that an alert may be presented in any suitable manner, examples of which were discussed above. Regardless of how an alert is generated, in step 510, the template report may be presented to the medical professional.

The processes 400 and 500 illustrate some examples of pre-populating medical reports with data from image study data and generating alerts based on image study data. It should be appreciated that the data used to pre-populate the template need not be the same as the data analyzed for determining whether to generate an alert. In some embodiments, alert generation may analyze a broader (or different) set of data than is used in pre-populating the report. This may enable alerts to be generated based on data not explicitly listed in the medical report.

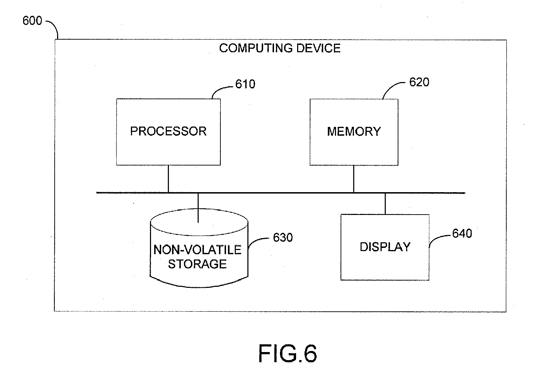

An illustrative implementation of a computing device 600 that may be used to implement one or more of the techniques described herein, e.g., to extract data and pre-populate a template medical report (e.g., in method 400 of FIG. 4) and/or to analyze data and generate alerts (e.g., in method 500 of FIG. 5), is shown in FIG. 6. Computing device 600 may include one or more processors 610 and one or more non-transitory computer-readable storage media (e.g., memory 620 and one or more non-volatile storage media 630). The processor 610 may control writing data to and reading data from the memory 620 and the non-volatile storage device 630 in any suitable manner, as the techniques described herein are not limited in this respect. To perform the functionality and/or techniques described herein, the processor 610 may execute one or more instructions stored in one or more of the computer-readable storage media (e.g., the memory 620, storage media, etc.), which may serve as non-transitory computer-readable storage media storing instructions for execution by the processor 610. Computing device 600 may also include any other processor, controller or control unit that routes data, performs computations, perform input/output (I/O) functionality, etc.

One or more programs that extract data, analyze data, pre-populate a template medical report, and/or generate alerts may be stored on one or more of the computer-readable storage media of computing device 600. Processor 610 may execute any one or combination of such programs that are available to the processor by being stored locally on computing device 600 or accessible over a network. Any other software, programs or instructions described herein may also be stored and executed by computing device 600. Computing device 600 may be any suitable device capable of executing one or more instructions.

In some embodiments, computing device 600 may have one or more input/output (I/O) components. For example, in some embodiments, computing device 600 may have a display 640, which may be a monitor or other type of display.

The above-described embodiments of the present invention can be implemented in any of numerous ways. For example, the embodiments may be implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments of the present invention comprises at least one non-transitory computer-readable storage medium (e.g., a computer memory, a USB drive, a flash memory, a compact disk, a tape, etc.) encoded with a computer program (i.e., a plurality of instructions), which, when executed on a processor, performs the above-discussed functions of the embodiments of the present invention. The computer-readable storage medium can be transportable such that the program stored thereon can be loaded onto any computer resource to implement the techniques and functionality discussed herein. In addition, it should be appreciated that the reference to a computer program which, when executed, performs the above-discussed functions, is not limited to an application program running on a host computer. Rather, the term computer program is used herein in a generic sense to reference any type of computer code (e.g., software or microcode) that can be employed to program a processor to implement the above-discussed techniques and functionality.

Various aspects of the present invention may be used alone, in combination, or in a variety of arrangements not specifically discussed in the embodiments described in the foregoing and are therefore not limited in their application to the details and arrangement of components set forth in the foregoing description or illustrated in the drawings. For example, aspects described in one embodiment may be combined in any manner with aspects described in other embodiments.

Also, embodiments of the invention may be implemented as one or more methods, of which an example has been provided. The acts performed as part of the method(s) may be ordered in any suitable way. Accordingly, embodiments may be constructed in which acts are performed in an order different than illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term).

The phraseology and terminology used herein is for the purpose of description and should not be regarded as limiting. The use of "including," "comprising," "having," "containing", "involving", and variations thereof, is meant to encompass the items listed thereafter and additional items.

Having described several embodiments of the invention in detail, various modifications and improvements will readily occur to those skilled in the art. Such modifications and improvements are intended to be within the spirit and scope of the invention. Accordingly, the foregoing description is by way of example only, and is not intended as limiting. The invention is limited only as defined by the following claims and the equivalents thereto.

## Claims

1. A computer implemented method of populating a medical report, the method comprising computer implemented steps of:
extracting data from a memory, the memory holding data from a medical imaging study, the extracted data comprising measurement data from the medical imaging study and/or calculation data from the medical imaging study; and
automatically populating at least one portion of the medical report with at least some of the extracted data, wherein the medical report comprises a medical professional's impression of the medical imaging study.

2. The computer implemented method of claim 1, wherein the extracting data from the medical imaging study comprises at least one of:
extracting measurement data from the medical imaging study, wherein the measurement data comprises data collected by at least one medical imaging device; and extracting calculation data from the medical imaging study, wherein the calculation data comprises data calculated based, at least in part, on data collected by at least one medical imaging device.

3. The computer implemented method of claim 1 or 2, further comprising:
automatically determining a type of the medical imaging study; and
automatically determining what data to extract from the medical imaging study based, at least in part, on the determination of the type of medical imaging study.

4. The computer implemented method of any of claims 1-3, wherein the medical imaging study comprises a DICOM Structured Report (SR) comprising binary data, and wherein the extracting data from the medical imaging study comprises converting the binary data into XML-formatted data and extracting the data from the XML-formatted data.

5. The computer implemented method of any of claims 1 to 4, wherein the at least one portion of the medical report is associated with a respective identifier identifying the type of data to be populated in said at least one portion, and the step of automatically populating a portion of the medical report comprises:
determining a data type of a portion of the extracted data; and
populating the portion of the medical report with said portion of the extracted data based on determining that the data type identified by the identifier associated with the portion of the medical report matches the data type of the portion of extracted data.

6. The computer implemented method of any of claims 1 to 4, wherein the at least one portion of the medical report is a merge field, the method comprising using a merge field module to locate and extract the data when said merge field is referenced in the medical report and automatically populating the merge field with the extracted data.

7. The computer implemented method of any of claims 1-6, further comprising:
analyzing at least one value of at least one data type from the medical imaging study, the at least one data type comprising at least one type of measurement data from the medical imaging study and/or at least one type of calculation data from the medical imaging study; determining whether the at least one analyzed data value is outside of a normal range for the at least one data type; and
during preparation of the medical report, generating an alert to the medical professional in response to determining that the at least one analyzed data value is outside of the normal range.

8. The computer implemented method of claim 7, wherein the extracted data comprises the at least one analyzed data value.

9. The computer implemented method of claim 7, wherein the at least one analyzed data value is not among the extracted data.

10. A system configured to populate a medical report, the system comprising:
at least one processor; and
at least one storage device storing computer-readable program instructions which, when executed by the at least one processor, performs the method of any of claims 1-9.

11. A computer program product comprising a computer readable medium having stored thereon computer-readable program instructions which, when executed by at least one processor, performs the method of any of claims 1-9.

12. A computer implemented method comprising computer implemented steps of:
analyzing at least one value of at least one data type from a medical imaging study held in a memory, the at least one data type comprising at least one type of measurement data from the medical imaging study and/or at least one type of calculation data from the medical imaging study;
determining whether the at least one analyzed data value is outside of a normal range for the at least one data type; and
during preparation of a medical report comprising a medical professional's impression of the medical imaging study, generating an alert to the medical professional in response to determining that the at least one analyzed data value is outside of the normal range.

13. The computer implemented method of claim 12, wherein analyzing at least one value of at least one data type comprises at least one of:
analyzing measurement data from the medical imaging study, wherein the measurement data comprises data collected by at least one medical imaging device; and analyzing at least one value of at least one data type comprises analyzing calculation data from the medical imaging study, wherein the calculation data comprises data calculated based, at least in part, on data collected by the at least one medical imaging device.

14. The computer implemented method of claim 12 or 13, wherein the medical imaging study relates to a patient, and wherein the normal range comprises a range of values corresponding to a determined normal range for a population comprising a plurality of subjects, and/or a determined normal range for the patient.

15. The computer implemented method of any of claims 12-14, further comprising:
automatically determining a type of the medical imaging study; and
automatically determining the at least one value of the at least one data type to analyze based, at least in part, on the determination of the type of medical imaging study.

16. The computer implemented method of any of claims 12-15, further comprising:
extracting data from the medical imaging study, the extracted data comprising measurement data from the medical imaging study and/or calculation data from the medical imaging study; and
automatically populating at least one portion of the medical report with at least some of the extracted data, wherein the medical report comprises the medical professional's impression of the medical imaging study.

17. A system comprising:
at least one processor; and
at least one storage device storing computer-readable program instructions which, when executed by the at least one processor, performs the method of any of claims 12 - 16.

18. A computer program product comprising a computer readable medium having stored thereon computer-readable program instructions which, when executed by at least one processor, performs the method of any of claims 12-16.
